Europäisches Patentamt

**European Patent Office**

Office européen des brevets

(11) Publication number: **0 141 484**
**A2**

(12) **EUROPEAN PATENT APPLICATION**

(21) Application number: **84303787.0**

(22) Date of filing: **05.06.84**

(51) Int. Cl.⁴: **C 12 N 15/00**
C 12 P 21/02, C 07 K 7/00
C 07 H 21/04

(30) Priority: **10.06.83 GB 8315980**

(43) Date of publication of application:
**15.05.85 Bulletin 85/20**

(84) Designated Contracting States:
**AT BE CH DE FR GB IT LI LU NL SE**

(71) Applicant: **BIOGEN N.V.**
**15 Pietermaai**
**Willemstad Curacao, Netherlands Antilles(NL)**

(72) Inventor: **Weissmann, Charles**
**Eschenhausstrasse 39**
**CH-8053 Zürich(CH)**

(72) Inventor: **Weber, Hans**
**Amwasser 113A**
**CH-8049 Zürich(CH)**

(74) Representative: **Bannerman, David Gardner et al,**
**Withers & Rogers 4 Dyer's Buildings Holborn**
**London, EC1N 2JT(GB)**

(54) Methods of producing hybrid DNA sequences and hybrid polypeptides and DNA sequences produced by them.

(57) A method for preparing hybrid DNA sequences characterized by the steps of (a) preparing a DNA fragment, or concatemer thereof, said fragment comprising in sequence one of the parental DNA sequences from which the hybrid DNA sequence is to be derived, an intact replicon such that the DNA fragment may be replicated in a host cell, and the other parental DNA sequence from which the hybrid DNA sequence is to be derived, the two parental DNA sequences having a sequence of at least three, and preferably at least five, nucleotides in common in the location where hybrid DNA sequence formation is sought and the direction of translation of the coding sequences in the two parental sequences being the same relative to each other in the DNA fragment; (b) selecting host cells that have been transfected with the desired hybrid DNA sequence and isolating said hybrid DNA sequence from them. The hybrid DNA sequences of this invention are useful in producing hybrid polypeptides having a variety of uses and biological activities.

METHODS OF PRODUCING HYBRID DNA SEQUENCES
AND HYBRID POLYPEPTIDES AND
DNA SEQUENCES PRODUCED BY THEM

TECHNICAL FIELD OF INVENTION

This invention relates to hybrid polypeptides, the DNA sequences coding for them and to methods of producing those DNA sequences and polypeptides. The methods of this invention are characterized by the steps of (a) preparing a DNA fragment or concatemer thereof, said fragment comprising in sequence one of the parental DNA sequences from which the hybrid DNA sequence is to be derived, an intact replicon such that the DNA fragment may be replicated in a host cell, and the other parental DNA sequence from which the hybrid DNA sequence is to be derived, the two parental DNA sequences having a sequence of at least three, and preferably at least five, nucleotides in common in the location where hybrid DNA formation is sought and the direction of translation of the coding sequences in the two parental sequences being the same relative to each other in the DNA fragment; and (b) selecting host cells that have been transfected with the desired hybrid DNA sequence and isolating said hybrid DNA sequence from them. Selection of the desired host cells may be facilitated by having each parental DNA sequence associated with a different antibiotic resistance marker and growing the transformed host cells on agar plates, containing both antibiotics. The hybrid DNA sequences

0141484

produced by the methods of this invention enable the production of novel hybrid polypeptides having a variety of uses and biological activities.

## BACKGROUND ART

Recent advances in recombinant DNA technology and molecular biology have enabled the isolation and identification of a variety of DNA sequences and genes that code for a large number of different polypeptides. For example, DNA sequences coding for the ten or more human α-interferons are described in C. Weissmann et al., Phil. Trans. R. Soc. Lond. B299, pp. 7-28 (1982). DNA sequences coding for human β-interferon and human γ-interferon are described in R. Derynck et al., Nature 285, pp. 542-547 (1980) and R. Devos et al., Nucleic Acids Research 10, pp. 2487-2501 (1982), respectively.

DNA sequences coding for non-interferon polypeptides are also available. They include DNA sequences coding for human insulin [D. Goeddel et al., Proc. Natl. Acad. Sci. USA 76, pp. 106-110 (1979)], rat insulin [L. Villa-Komaroff et al., Proc. Natl. Acad. Sci. USA 75, pp. 3727-3731 (1978)], human growth hormone [D. Goeddel et al., Nature 281, pp. 544-548 (1979)] and bovine growth hormone [W. Miller et al., DNA 1, pp. 37-50 (1981); W. Miller et al., Endocrinology 107, pp. 851-854 (1980)]. DNA sequences coding for a variety of viral antigens are also available. These include DNA sequences encoding the viral antigens of hepatitis B virus [M. Pasek et al., Nature 282, pp. 575-579 (1979); C. Burrell et al., Nature 279, pp. 43-47 (1979)] and the DNA sequences encoding the viral antigens of foot and mouth disease virus [H. Küpper et al., Nature 289, pp. 555-559 (1981)].

Certain hybrid DNA sequences consisting of parts of two or more of the above-described DNA sequences are also available. These hybrids are conventionally prepared by using restriction sites that occur in the various DNA sequences and ligating two restricted fragments together at a common restriction site or by blunt-end ligation of different restriction fragments to produce the hybrid DNA sequence. These hybrid DNA sequences may then be employed to transform appropriate host cells and to produce the hybrid polypeptide coded for by them. For example, hybrid DNA sequences of human α1 and α2 interferon, and the hybrid interferons coded for by them, have been prepared using this restriction and ligation method [M. Streuli et al., Proc. Natl. Acad. Sci. USA 78, pp. 2848-2852 (1981), R. Lawn et al., Nucleic Acids Res. 9, pp. 1045-1052 (1981)].

Depending on the particular construction and components of these hybrid interferons, they may display similar or vastly different specificities, activities and properties than the parental polypeptides from which they are derived. For example, although the specific activity of human α1 interferon is comparatively low in human cells and comparatively high in mouse cells, hybrids of α1 and α2 carrying the N-terminal half of α2 have high activity in human cells.

Although these prior methods have permitted the formation of certain hybrid DNA sequences and their use in the synthesis of certain hybrid polypeptides, they are very limited in their application. For example, hybrid formation by these prior methods requires the existence of a restriction site in both DNA sequences at the point where hybrid formation is desired. More usually, these methods require digestion of the DNA sequences from a convenient restriction site to the point where hybrid formation is desired and determination of when the digestions have reached the correct nucleotide. This is a very laborious and time consuming undertaking. Moreover, in DNA sequences that are closely related like the α-interferons, in order to avoid deleting or adding nucleotides in one or both of the DNA sequences, the restriction sites or digestions have to be in homologous portions of the DNA sequences. Such convenient restriction sites, digestions and homologies are plainly often not available. Furthermore, even in the few instances when such restriction sites are available, the number of possible hybrids able to be made from them is very small and their preparation very time consuming.

Therefore, there is a need in the recombinant DNA art to develop new methods for forming hybrid DNA sequences that do not depend on the chance availability of appropriate restriction sites in the two DNA sequences which one seeks to combine or in sequential deletions from one or both of those sites in order to produce products of modified use, activity and specificity, as compared to the polypeptides coded for by their parental DNA sequences.

## DISCLOSURE OF THE INVENTION

This invention solves the above-described problems and avoids the disadvantages that beset prior methods of hybrid DNA sequence formation. It provides for the first time novel hybrid polypeptides, the DNA sequences that code for them and methods of producing those DNA sequences and polypeptides.

The novel hybrid DNA sequences of this invention are characterized by at least two connected sequences of nucleotides, the first sequence of nucleotides being connected to the second sequence of nucleotides in their original reading frames and so as to maintain constant that reading frame in each sequence through a DNA sequence of at least three, and preferably at least five, nucleotides that was common to or inserted into both parental DNA sequences at the location where hybrid formation was sought. These novel DNA sequences encode the hybrid polypeptides of this invention and enable their production in appropriately transformed hosts.

The methods of this invention enable the construction of those hybrid DNA sequences and polypeptides in a simple and efficient manner.

In one embodiment of the method of this invention, where the two parental DNA sequences from which the hybrid DNA sequence is to be derived originally have a sequence of at least three, and preferably at least five, nucleotides, and more preferably at least 8, and most preferably at least 13 nucleotides, in common, in the location where hybrid DNA sequence formation is sought, the method comprises the steps of (a) preparing a DNA fragment or more usually a concatemer thereof, said fragment comprising in sequence one of the parental DNA sequences from which the hybrid DNA sequence is to be derived, an intact replicon such that the DNA fragment may be replicated in a host cell, and the other parental DNA sequence from which the hybrid DNA sequence is to be derived, the two parental DNA sequences having a sequence of at least 3, and preferably at least 5, nucleotides in common in the location where hybrid DNA formation is sought and the direction of translation of the coding sequences in the two parental sequences in said fragment being the same relative to each other; and (b) selecting host cells that have been transfected with the desired hybrid DNA sequence and isolating said hybrid DNA sequence from them.

In another embodiment of the method of this invention, where the two parental DNA sequences from which the hybrid DNA sequence is to be derived do not originally have a sequence of at least three, and preferably at least five, nucleotides in common at the location where hybrid DNA sequence formation is sought, the method comprises the steps of (a) preparing a linear DNA fragment or a concatemer thereof, said fragment comprising in sequence one of the parental DNA segments from which the hybrid DNA sequence is to be derived, an intact replicon such that the fragment may be replicated in a host cell, the other of the parental DNA sequences from which the hybrid DNA sequence is to be derived, the direction of translation of the coding sequences in the two parental sequences being the same relative to each other in the DNA fragment; (b) forming in one of said parental DNA sequences at the location where said hybrid formation is sought a sequence of at least three, and preferably at least five, nucleotides, said nucleotide sequence being identical to and in the same reading frame as a DNA sequence at the location where said hybrid formation is sought in said other parental DNA sequence; and (c) selecting host cells that have been transfected with the desired hybrid DNA sequence and isolating said hybrid DNA sequences from them.

It should of course be understood that in this embodiment of our invention the formation of the common nucleotide sequence in one or the other DNA sequence may be done prior to preparing the DNA fragment. Moreover, it should be also understood that the necessary homology in the two parental DNA sequences could also be provided by inserting into both DNA sequences the same or similar sequences so as to build the required sequence homology into both sequences before or after preparing the DNA fragment.

In the more preferred embodiment of this invention, each of the parental DNA sequences is also associated with a different antibiotic resistance marker. In this preferred embodiment, selection of the desired host cells may be facilitated by growing the transformed host cells on agar plates containing both antibiotics. This preferred method of selection is very useful in this invention from a practical standpoint because the yield of desired recombinants or hybrid DNA sequences is low and even a minute contamination with one or the other parental DNA sequences or with DNA sequences resulting from the ligation of two identical parental DNA sequences may result in a high level of unwanted transformants that would be difficult to screen in order to select the desired hybrid without such dual antibiotic resistance.

In still another embodiment of this invention, the circularization and ligation step to form the hybrid

0141484

DNA sequence is done in vitro, leaving only heteroduplex repair to take place in the host cell after its transfection with that hybrid DNA sequence. In this less preferred embodiment, the ends of the DNA fragment, or concatemer thereof, are rendered single-stranded before in vitro circularization and ligation.

The DNA sequences of this invention are characterized by at least two connected sequences of nucleotides, each connected DNA sequence being derived from a different parental DNA sequence, the first sequence of nucleotides being connected to the second sequence of nucleotides in the original reading frame and so as to maintain constant that reading frame in each sequence through a DNA sequence of at least three, and preferably at least five, nucleotides that was common to or inserted into both parental DNA sequences at the location where hybrid formation was sought. These DNA sequences enable the production of novel proteins and polypeptides having a variety of uses and biological activities.

## BRIEF DESCRIPTION OF FIGURES

Figure 1 is a schematic outline of one embodiment of a method of preparing the hybrid DNA sequences of this invention.

Figure 2 is a schematic outline of another embodiment of an in vitro method of preparing the hybrid DNA sequences of this invention.

Figure 3 is a schematic outline of a third embodiment of a method of preparing the hybrid DNA sequences of this invention.

Figure 4 is a schematic outline of the embodiment of this invention depicted in Example I.

Figure 5 is a schematic outline of the construction of pM11Kan-Xba.

Figure 6 displays the nucleotide sequences of IFN-α1 and IFN-α2 and 11 crossover regions A-K where

0141484

hybrids were isolated using one embodiment of the methods of this invention.

Figure 7 is a graphical display of the antiviral activities of various α2-α1 hybrid interferons on different target cells.

Figure 8 is a schematic outline of one embodiment of a method of this invention for constructing α2-β hybrid interferons.

Figure 9 displays the nucleotide sequences of IFN-β and IFN-α2 aligned by computer for "maximum sequence homology" and four crossover regions F3, F8, F10 and F15 where hybrids were isolated using one embodiment of the methods of this invention.

Figure 10 is a schematic outline of one embodiment of a method of this invention for constructing human α2-mouse α2 hybrid interferons.

## BEST MODE OF CARRYING OUT THE INVENTION

In order that the invention herein described may be more fully understood, the following detailed description is set forth.

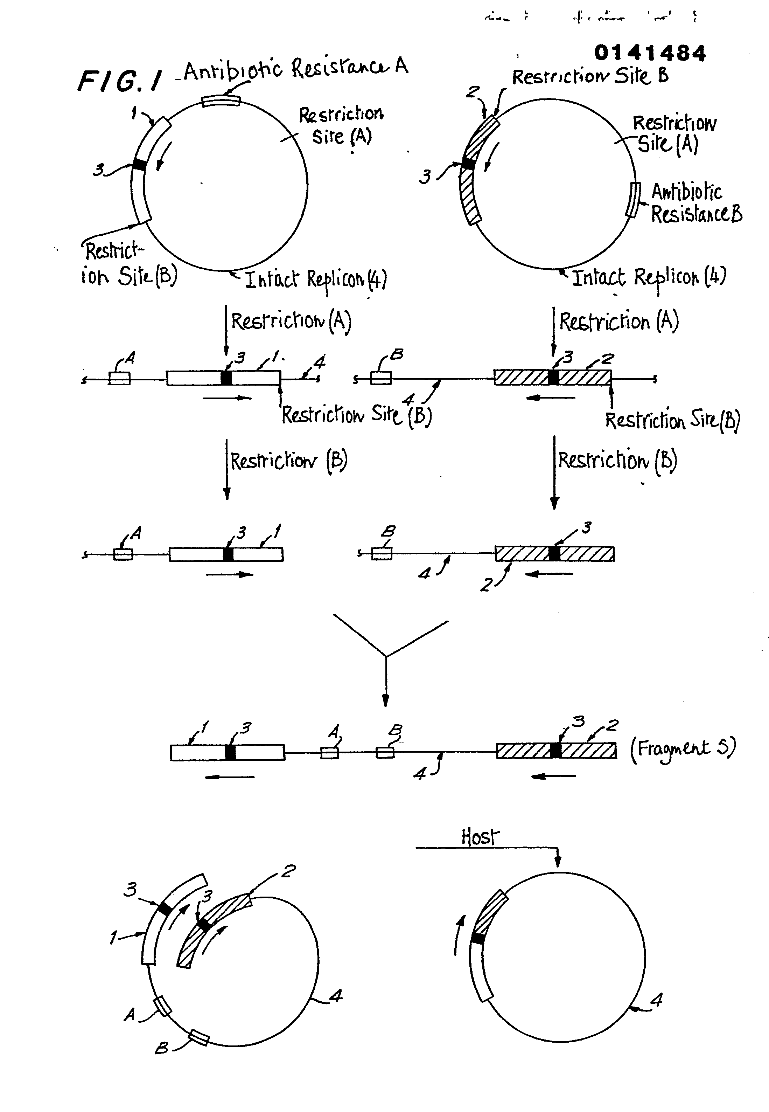

Referring now to Figure 1, we have depicted one preferred embodiment of a method according to this invention for preparing the novel hybrid DNA sequences of this invention. The two parental DNA sequences depicted in Figure 1, sequences 1 and 2, from which the hybrid DNA sequence is derived, have a sequence (3) of at least three, and preferably at least five, and more preferably at least 8, and most preferably at least 13 nucleotides, in common in the location where hybrid DNA sequence formation is sought.

As depicted in Figure 1, parental sequences 1 and 2 are part of plasmids or other cloning vehicles. However, it is to be understood that the parental DNA sequences useful in the methods of this invention may be derived from any source of DNA sequences provided that upon combination they form a DNA fragment (fragment 5 in Figure 1), or a concatamer thereof, said fragment comprising in sequence one of the parental DNA sequences, an intact replicon (4 in Figure 1) such that the fragment may be replicated in a host cell, and the other parental DNA sequence, the two parental DNA sequences having a sequence of at least three, and preferably at least five, nucleotides in common in the location where hybrid DNA formation is sought and the direction of translation of coding sequences in the two parental sequences being the same relative to each other in the DNA fragment. Such sources of DNA sequences include, for example, natural sources, synthetic sources, semisynthetic sources, cDNA libraries and combinations of them.

In the embodiment depicted in Figure 1, DNA fragment 5 comprises parental DNA sequences 1 and 2 and intact replicon 4. It also comprises antibiotic resistance markers A associated with the first parental DNA sequence and antibiotic resistance markers B associated with the second parental DNA sequences.

DNA fragment 5 in that embodiment was prepared by first linearizing the plasmids carrying parental DNA sequences 1 and 2 using a restriction site located outside of the respective parental DNA sequences. The restriction site used for such linearization need not be the same in both plasmids, although preferably the same site is employed for ease of combination of the two resulting fragments through the compatible restriction residues. However, it is to be understood that various means of combining the two fragments that do not require the same restriction residues may also be employed in the methods of this invention. These means, for example, include tailing, bluntending, fill-in and synthetic linkers, as well as use of two different restriction sites which yield the same overhanging ends, for example, BglII and BamHI.

In the embodiment depicted in Figure 1, the linearized plasmids were next restricted (Restriction Sites B) to remove that portion of the linearized plasmid located before the start of parental DNA sequence 1 and that portion of the linearized plasmid located after the end of parental DNA sequence 2. The resulting DNA fragments were then combined to form Fragment 5, or a concatemer thereof, said fragment having only one replicon and the direction of translation of the two coding sequences from the parental DNA sequences being the same relative to each other in the DNA fragment. Fragment 5 also carries antibiotic resistance markers A and B, respectively associated with the two different parental DNA sequences to facilitate selection of the desired fragment construction.

Again, it is to be understood that the particular sequence of steps employed in the embodiment of this invention depicted in Figure 1 is not required to construct DNA sequence 5 employed in this invention to prepare the novel hybrid DNA sequences of this invention. For example, although it is preferred that the DNA sequence 5 start at one end with the first parental DNA sequence and end at the other end with the other parental sequence, DNA sequences that begin and/or end outside of the parental DNA sequences may also be used in the methods of this invention, provided the additional nucleotide sequences do not occur elsewhere in the sequence chosen. DNA sequences that begin and/or end within the parental DNA sequences are also useful in this invention. All that is required for the method of this invention is that the DNA sequence 5 comprise in sequence one parental DNA sequence (1), an intact replicon (4) and the other parental DNA sequence (2), the two parental DNA sequences having a sequence of at least three, and preferably at least five, nucleotides in common in the location where hybrid DNA formation is sought and the direction of translation of the coding sequences in the two parental DNA sequences being the same relative to each other in the fragment.

In the embodiment depicted in Figure 1, circularization of DNA sequence 5 and overlap of the homologous sequences of nucleotides in the location where hybrid formation is sought in both parental DNA sequences is effected in vivo by the host cell which is transfected by the DNA sequence. The host cell used for this transfection should be recombination proficient, and preferably be rec A+. In the embodiment depicted in Figure 1, the host cell effects combination of the parental DNA sequences within that region of DNA sequence homology and heteroduplex repair to form a vector or plasmid that contains the desired hybrid DNA.

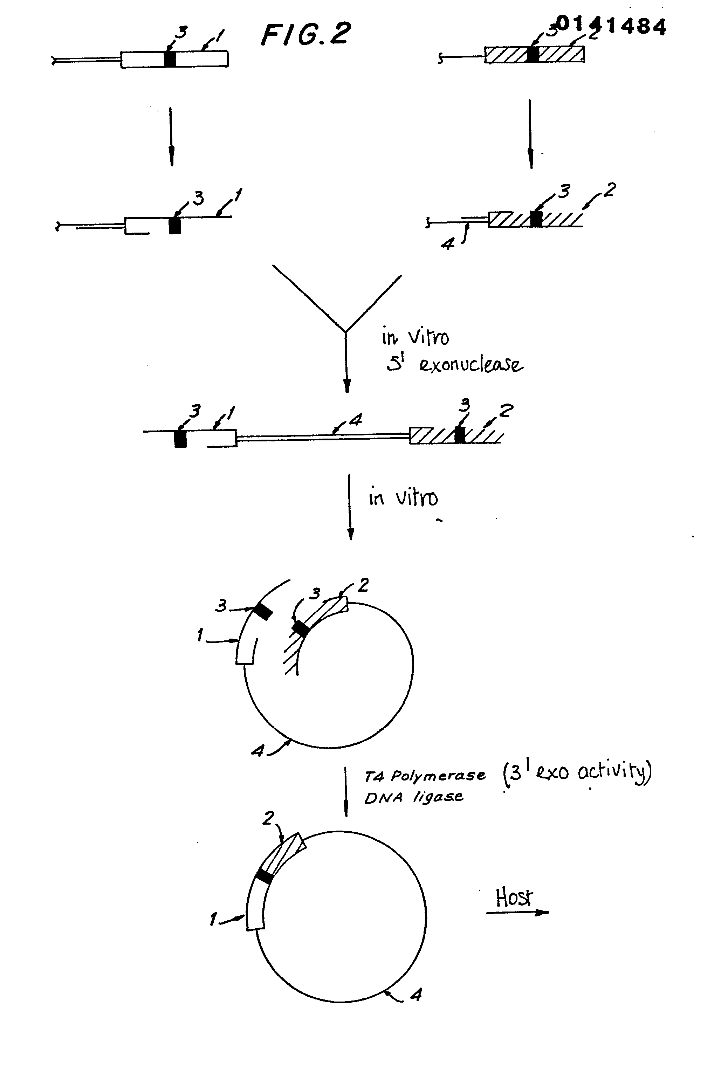

It should, however, also be understood that recircularization and recombination of the DNA sequences may also be done in vitro using conventional means and the host employed only to effect heteroduplex repair. See, for example, Figure 2. However, because it involves more biochemical steps, in vitro combination is less preferred in the practice of this invention.

In the preferred embodiment of this invention, depicted in Figure 1, the parental DNA sequences had the required homology pre-existing at the location where hybrid DNA sequence formation was desired. Such parental DNA sequences include, for example, the fifteen or more human leukocyte interferons, the various animal leukocyte interferons, including mouse, bovine, porcine, equine and chicken, human fibroblast interferon, which is characterized by stretches of homology of up to 13 nucleotides as compared to the leukocyte interferons, animal fibroblast interferons, human immune interferon and animal immune interferons. They may also include various related lymphokines and the antigens to the various strains and serotypes of foot and mouth disease virus.

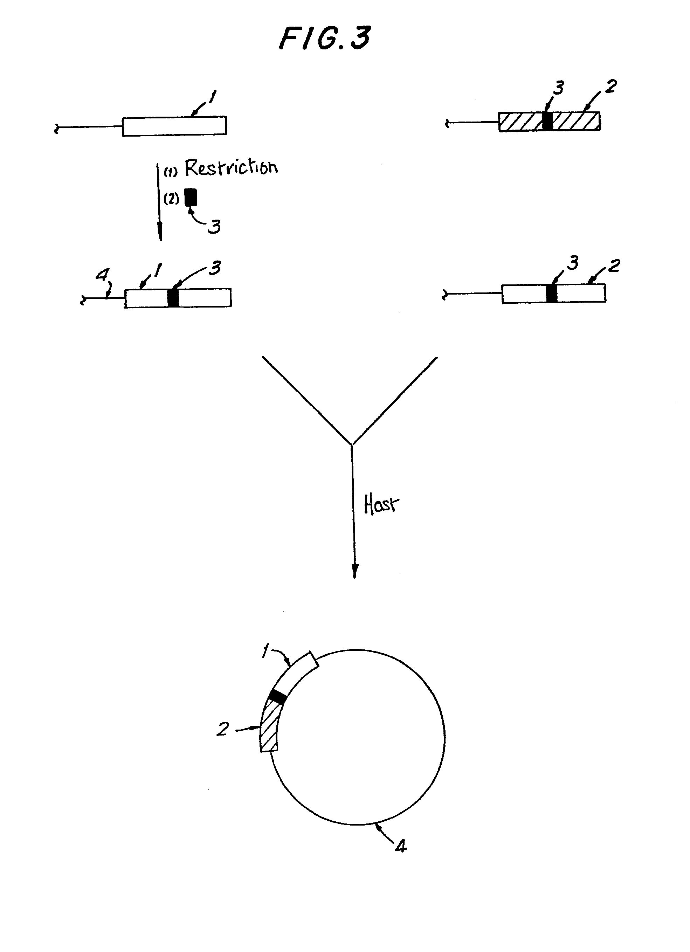

In a less preferred embodiment of this invention, the parental DNA sequences do not have the required homology. Accordingly, in that embodiment, which is depicted in Figure 3, the required homology is added to one or both of the parental DNA sequences in vitro by adding a sequence of nucleotides that corresponds to the sequence of nucleotides in the other parental DNA sequence or some predetermined sequence at the location where hybrid DNA sequence formation is sought. After providing the required homology to the two parental DNA sequences, the steps in the method of this invention, previously described, may be usefully employed in this embodiment to produce the novel hybrid DNA sequences of this invention. Therefore, the method of this invention is applicable to any parental DNA sequence.

Having prepared the novel hybrid DNA sequences of this invention by the methods described above, those sequences may then be employed to produce the novel polypeptides coded for by them. Various known methods and hosts may be employed to express these hybrid DNA sequences to produce those novel polypeptides. For example, the hybrid DNA sequence may be isolated from the plasmid produced in the above-described method, inserted into various known expression vectors under the control of various known expression control sequences and employed to transform various hosts, which upon culturing will produce the desired polypeptides.

Expression control sequences are sequences of nucleotides that control and regulate the expression of DNA sequences when operatively linked to those sequences in expression vectors. Those useful in expressing the novel hybrid DNA sequences of this invention include, but are not limited to, the lac system, trp system, the major operator and promoter regions of phage λ, the control regions of fd coat protein, the β-lac system, the TAC system, the TRC System, and other sequences known to control the expression of genes of prokaryotic or eukaryotic cells or their viruses or·combinations thereof.

Hosts useful in accordance with this invention for preparing the hybrid DNA sequences of this invention in vivo and for expressing those hybrid DNA sequences include various strains of E.coli, e.g., E.coli HB101, E.coli 1776, E.coli 2282, E.coli MRCI, E.coli SK1592 and

E.coli 803, Pseudomonas, Bacillus, including Bacillus subtilis and Bacillus stearothermophilus, yeasts and other fungi, animal, including Chinese Hamster ovary cells, or plant hosts and human cells in culture. For transfection to produce the hybrid DNA sequence, the strains should be recombination proficient and more preferably be rec A+. Of course not all host/expression vector combinations may be equally efficient for the formation of or expression of a particular hybrid DNA sequence. However, the particular selection of a host/vector combination may be made by those of skill in the art without departing from the scope of this invention.

Similarly, methods of inserting the hybrid DNA sequence into the above-described expression vectors, transforming the above-described host or culturing those hosts may be chosen from among various well-known methods without departing from the scope of this invention.

The novel polypeptides produced using the hybrid DNA sequences of this invention have a variety of uses. For example, the hybrid α-interferons produced by this invention have various activities in certain cells and various specific activities. Accordingly, such hybrids are useful in modifying the anti-viral and anti-cancer activities of the parental interferons for use in specific cells or against specific diseases. In like manner, hybrid proteins from the antigens of foot and mouth disease are useful in di- or multivalent vaccines against various strains and serotypes of foot and mouth disease virus.

In order to describe this invention so that it may be more clearly understood, the following examples are set forth. These examples are for the purpose of illustration and any specific enumeration therein should not be construed as a limitation of this invention.

The enzymes that we used in these examples were purchased from New England Biolabs Inc. We purchased the labeled nucleotides from Amersham. And, we acquired the E.coli strains as follows: SK1592 from Dr. S. Kushner, University of Georgia; 803 from Dr. K. Murray, University of Edinburgh; and $CaCl_2$-treated SK1592 was a gift from M. Streuli.

We prepared the plasmids containing the parental α1 and α2 interferon genes by modifications of published methods [T. Currier et al., Anal. Bioch. 76, pp. 431-441 (1976); D. Holmes et al., Anal. Bioch. 114, pp. 193-197 (1981)].

## EXAMPLE 1

Example 1 demonstrates the formation of hybrid DNA sequences coding for human α1/α2 hybrid interferons in vivo using the recombination machinery of E.coli and the production of such hybrid proteins in E. coli. The DNA sequences for α1 and α2 human interferon are described, for example, in M. Streuli et al., "At Least Three Human Type α Interferons: Structure of α2", Science, 209, pp. 1343-47 (1980). See also European patent application 32134 and clones deposited and made available in connection therewith.

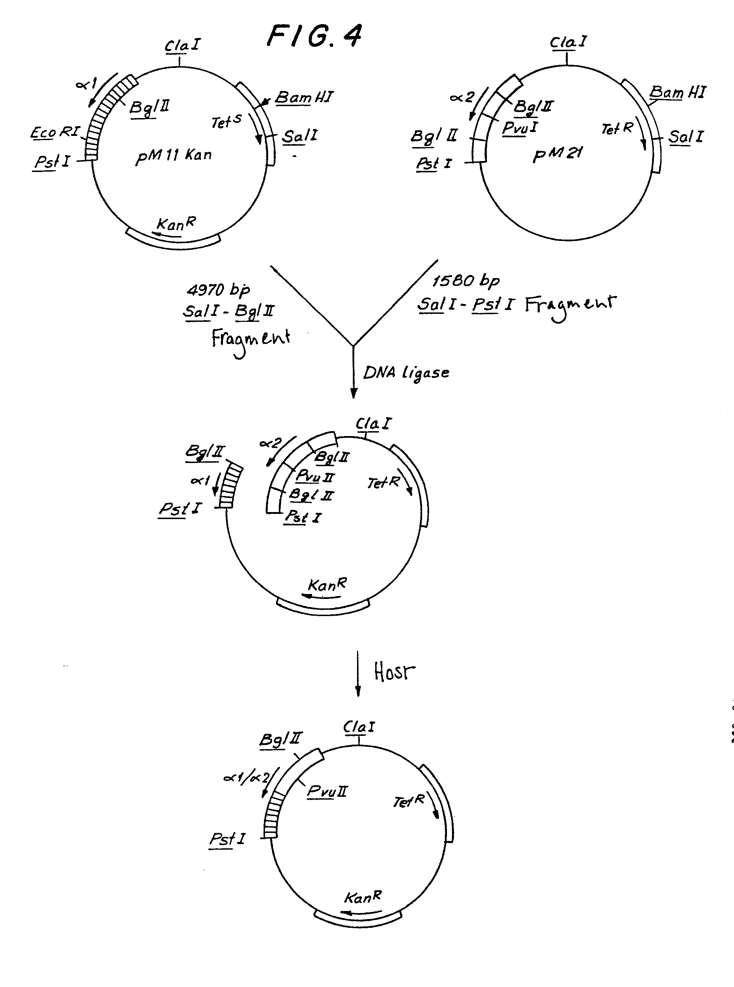

We constructed plasmid pM21 (constructed for high expression of interferon α2 in E.coli) by fusing the DNA sequence coding for mature α2 interferon to the initiation codon of the β-lactamase gene of plasmid pBR322. In that construction the interferon coding sequence and its 3'-non-coding sequence replaced the β-lactamase coding sequence down to the PstI site, where it was linked to the β-lactamase sequence by a poly(dGC) sequence. The remainder of the plasmid was identical to pBR322, including its intact tetracycline resistance coding region (Figure 4). See also European patent application 32134; ATCC 31769.

We constructed the other parental plasmid, pMllkan, by inserting the α1 interferon coding sequence into a derivative of pBR322, in substantially the same way that we inserted the coding sequence for α2 interferon

0141484

into pBR322 to obtain the pM21 plasmid (Figure 4). More specifically, we first prepared a derivative of pBR322 that included the genes coding for resistance to tetracycline, ampicillin and kanamycin (pBR322kan), as described below. First, we partially digested pBR322 with HaeII in the presence of 50 µg/ml ethidium bromide to yield mostly full-size linear molecules. We then ligated these fragments to an equivalent amount of a 1430 bp fragment prepared by complete HaeII digestion of a derivative of pCRI (a gift of M. Billeter). This fragment contained the gene coding for kanamycin resistance. We then selected transformants for resistance to kanamycin and ampicillin, and subsequently for resistance to tetracycline. For some reason, the simultaneous selection for resistance to all three antibiotics did not result in any surviving transformants.

Restriction mapping of our selected transformants demonstrated that in our construction the kanamycin resistance gene, contained in the 1430 bp HaeII fragment of pCRI, had been inserted into the HaeII site of pBR322 at position 2352 (Figure 4).

We then prepared pM11kan by combining the following DNA sequences: (1) the 4400 bp PstI-SalI fragment of the above-described kanamycin resistant pBR322 derivative (pBR322kan), (2) the 1000 bp ClaI-PstI fragment of pM11, containing the interferon α1 coding sequence, and (3) the about 800 bp ClaI-SalI fragment of a pBR322 derivative (pBR322Bam$^R$), containing a lambda DNA fragment linked by poly (dG:dC) tracts inserted in the BamHI site where it inactivated the gene coding for tetracycline resistance present in that fragment. We had isolated the three fragments, described above, by electrophoresis on low-melting agarose gels (Sea Plaque agarose, FMC Corporation) and used the gel slices after melting them at 65°C directly for ligation. We selected transfectant colonies for kanamycin resistance and then on the basis of their tetracycline and ampicillin sensitivity. We confirmed the

0141484

structure of the desired pM11kan by restriction mapping (Figure 4).

To prepare the hybrid DNA sequences of this invention, we linearized pM11kan and pM21, and combined fragments thereof to prepare a DNA fragment, or more usually a concatemer thereof, said fragment containing both parental DNA sequences and an intact replicon. The fragment also contains one antibiotic resistance marker (Kanamycin) and a portion of another antibiotic resistance marker (tetracycline) associated with the α1 coding sequence and the portion of the tetracycline resistance marker that completes it associated with the α2 coding region. As a result, selection of the desired hybrid DNA is facilitated because only correctly constructed fragments will display both antibiotic resistances.

To prepare this DNA fragment, we restricted pM21 with SalI and PstI and isolated the 1580 bp SalI-PstI fragment of pM21 (Figure 4). We also restricted pM11Kan with SalI and BglII and isolated the 4970 bp SalI-BglII fragment of pM11kan. We isolated these fragments from the digests by electrophoresis on low melting agarose (1.2% and 0.8%, respectively) in 40 mM Tris-acetate, 1 mM EDTA (pH 7.8). We then ligated the two fragments together at 16°C for 18 h in a 20 μl reaction mixture, containing 20 mM Tris-HCl (pH 7.8), 10 mM $MgCl_2$, 10 mM DTT, 0.5 mM ATP, 10-20 fmol DNA fragments from melted slices of the agarose (2-5 μl aliquots of agarose slices method at 65°C, final agarose concentration 0.35%) and DNA ligase (400 units, obtained from New England Biolabs).

The resulting DNA fragment (Figure 4) comprised in sequence the DNA sequence encoding mature α2 interferon, the DNA sequences encoding tetracycline resistance and kanamycin resistance, an intact replicon from pBR322 and the DNA sequence encoding the COOH-terminal part of α1 interferon. Moreover, the parental α1 and α2 DNA sequences were in the correct translational orientation relative to each other in the DNA fragment to allow parallel overlap of homologous sequences of at least 3 nucleotides at the location where hybrid formation was sought. Accordingly, circularization of this DNA sequence by the methods of this invention produced hybrid DNA sequences encoding the amino terminal of α2 and the carboxy terminal of α1, with the location of hybrid formation being downstream of the BglII site in the α1 coding region.

In order to circularize the DNA fragment and to form the desired hybrid DNA sequences, we transfected the DNA fragment into calcium chloride-treated E.coli SK1592 [prepared according to Cohen et al., Proc. Natl. Acad. Sci. USA, 68, pp. 2110-2114 (1972), and stored at -80°C in a solution of 50 mM $CaCl_2$ in 20% glycerol]. We employed 100 µl transfection mixtures, containing 2.5 µl aliquots of the ligated DNA, 10 mM $CaCl_2$, 10 mM $MgCl_2$, 10 mM Tris-HCl (pH 7.5) and 65 µl of a suspension of the $CaCl_2$-treated E.coli cells. We then maintained the resulting suspensions at 0°C for 15 min and then at 42°C for 2 min, diluted them with 1 ml of L-broth and incubated them for 90 min at 37°C with shaking.

We then spread aliquots of the mixture of transformed cells on agar plates, containing L-broth, kanamycin (50 µg/ml) and tetracycline (20 µg/ml). This selection process ensured that only cells transformed with DNA sequences containing elements of both parental DNA fragments linked correctly at the SalI site gave rise to viable colonies because in our selection the proximal portion of the tetracycline resistance region has to be provided by the α2-containing fragment whereas the distal portion of the tetracycline resistance region as well as the kanamycin resistance gene must originate from the α1-containing fragment (Figure 4).

In two separate experiments, we obtained 3 and 47 positive colonies, respectively, from transfection of portions (containing 1-2 fmol fragments) of the above ligation mixtures. The small number of colonies that we obtained in the first experiment may be due to the fact that the agarose-containing ligation mixture we used in that experiment had been melted at 65°C once before and stored subsequently at -25°C for several days prior to its use.  In subsequent experiments the number of colonies varied between 1600 and 90000 per pmol DNA fragments.

We isolated plasmids from the cultures selected above by well-known methods and analyzed the plasmids by restriction and DNA sequence analysis [L. Guo et al., Nucl. Acids Res., 10, pp. 2065-2084 (1982)].  We have summarized the results of our analyses in Table I.

With the exception of clone 12.1, which contained a deletion, the clones analyzed represented correctlyrecombined hybrid DNA sequences without any gaps or insertions.  Our conclusion is supported by (1) the distances between the BglII sites and the flanking restriction sites on the 5' and 3' sides of the interferon insert (Figure 4), (2) sequence determinations of relevant portions of 21 plasmids, and (3) the fact that the bacterial cultures containing these recombinants, except for untested clone 12.1, produced interferon activities similar to the parental IFN-α2, when tested on human ccl23 cells.

Among the 21 sequenced clones, we identified seven different crossover regions with the crossover region corresponding to the longest stretch of uninter-rupted homology between the α1 (396-452 bp) and α2 (393-449 bp) sequence, being represented no less than 12 times. Moreover, these seven sequences code for five different α2-α1 hybrid interferons, only one of which was identical to the α2-α1 hybrid interferon previously-obtained by Streuli et al., supra, using conventional genetic engineering techniques.

Plasmid DNA extracted from the cultures of six of the selected colonies (3, 3-4, 102, 105, 107 and 110) showed heterogeneity with regard to the second BglII site

0141484

and plasmid DNA from one of these colonies (107) also showed heterogeneity with regard to the EcoRI site. These heterogeneities may be a result of the segregation of incompletely-repaired heteroduplex strands or, alternatively, the result of transfection by several copies of recombination-competent DNA, presumably covalently linked to concatenates.

0141484

## TABLE I*

| Clone No. | No. of BglII sites | No. of PvuII sites | No. of EcoRI sites | Region of Recombination by Sequencing (bp no. (2) to bp no. (1)) |
|---|---|---|---|---|
| 12.1 | 0 | n.d. | n.d. | n.d. |
| 12.2 | 1 | 2 | 1 | 259-285 |
| 12.3 | 2 | 2 | 1 | 454-461 |
| 1 | 1 | 2 | 1 | 393-452 |
| 2 | 2 | 2 | 0 | n.d. |
| 3 | 1+2(mix) | 2 | 1 | n.d. |
| 3-2 | 1 | 2 | 1 | 393-452 |
| 3-4 | 1+2(mix) | 2 | 1 | n.d. |
| 4 | 1 | 2 | 1 | 311-318 |
| 5 | 1 | 2 | 1 | 311-318 |
| 6 | 1 | 2 | 1 | 393-452 |
| 7 | 2 | 2 | 0 | n.d. |
| 8 | 1 | 2 | 1 | 393-452 |
| 101 | 1 | 2 | 1 | 393-452 (372-394 not strictly excluded) |
| 102 | 1+2(mix) | 2 | 1 | n.d. |
| 102-1 | 1 | 2 | 1 | 393-452 |
| 102-2 | 2 | 2 | 1 | 460-482 |
| 103 | 1 | 2 | 1 | 393-452 (372-394 not strictly excluded) |
| 104 | 1 | 2 | 1 | 360-452 (probably 393-452) |
| 105 | 1+2(mix) | 2 | 1 | n.d. |

---

\* Abbreviations used in Table I:
    "n.d."      not done

0141484

TABLE I (cont.)

| Clone No. | No. of BglII sites | No. of PvuII sites | No. of EcoRI sites | Region of Recombination by Sequencing (bp no. (2) to bp no. (I)) |
|---|---|---|---|---|
| 105-1 | 2 | 2 | 1 | 460-482 |
| 106 | 2 | 2 | 0 | n.d. |
| 107 | 1+2(mix) | 2 | 0+1(mix) | n.d. |
| 107-1 | 1 | 2 | 1 | 339-350 |
| 107-4 | 2 | 2 | 0 | n.d. |
| 108 | 1 | 2 | 1 | 393-452 |
| 109 | 1 | 2 | 1 | 311-318 |
| 110 | 1+2(mix) | 2 | 1 | n.d. |
| 110-1 | 1 | 2 | 1 | 393-452 |
| 110-2 | 2 | 2 | 1 | 460-482 |
| 111 | 1 | 2 | 1 | 393-452 |
| 112 | 1 | 2 | 1 | 393-452 |

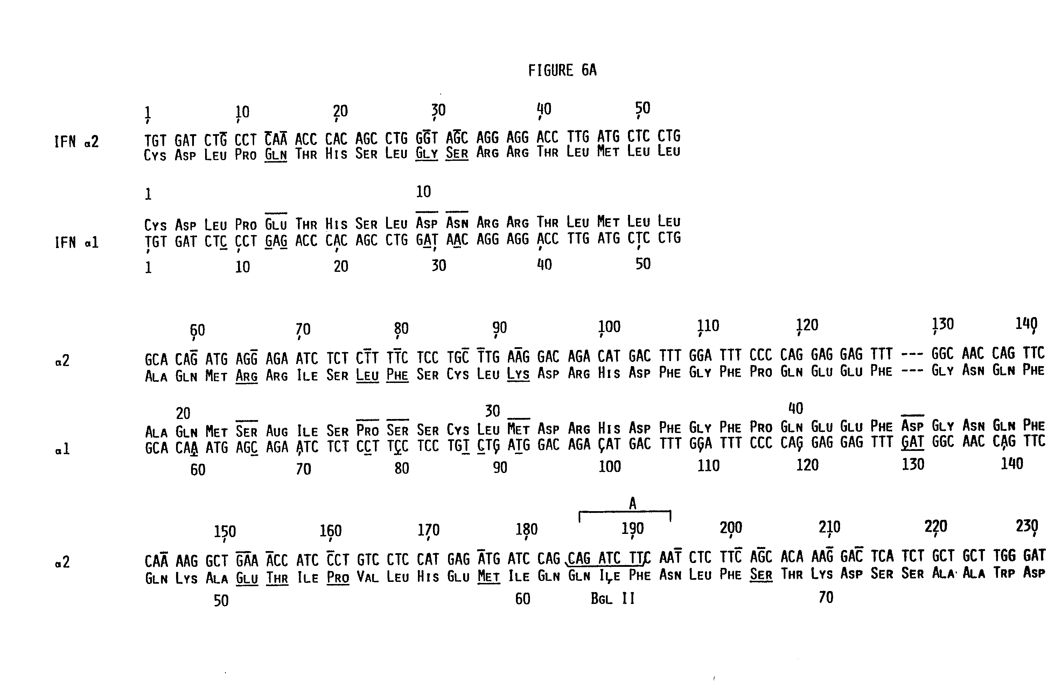

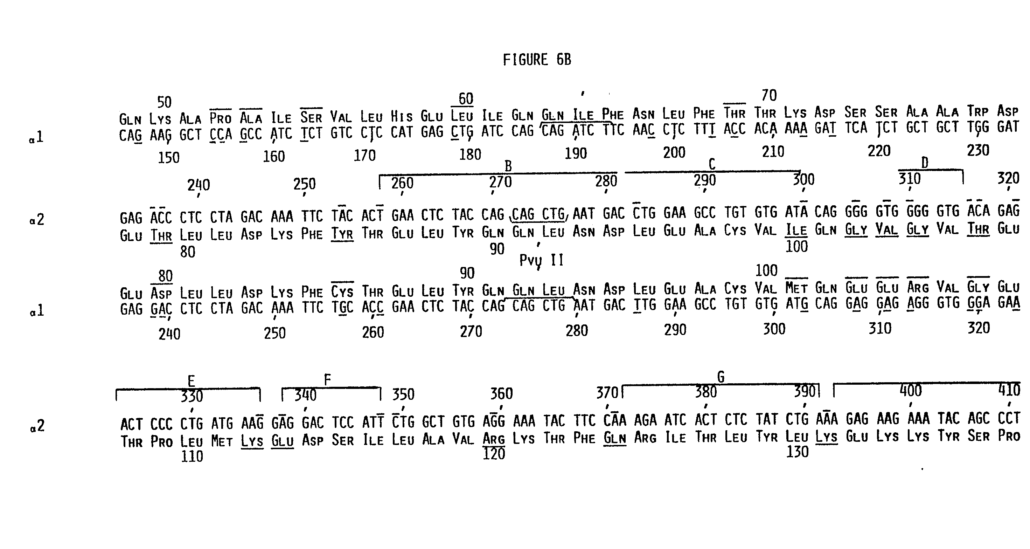

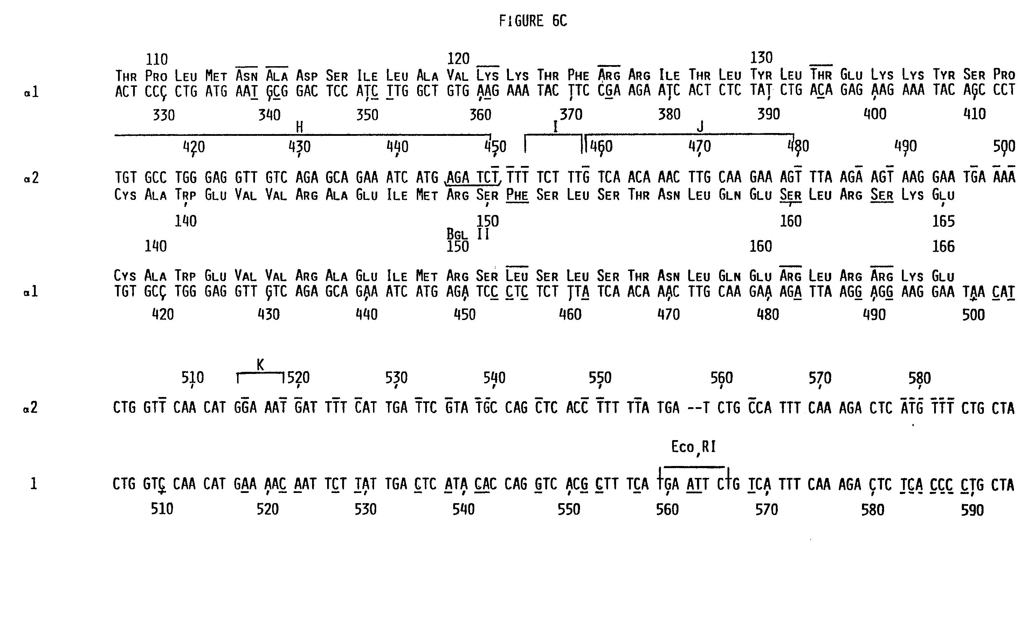

Including additional experiments using the method of this Example I, we have isolated recombinant α2-α1 coding sequences having 11 different crossover regions. See Figure 6. These 11 regions are designated A through K in Figure 6. One of these crossover regions (K) had a homology of only three nucleotides, demonstrating that three nucleotides was sufficient homology between the two parental DNA sequences for recombination using the methods of this invention.

The 11 different recombinants encoded 9 different hybrid interferons. These arose from recombinants with crossovers in regions A, D, E, F, G, H and I/J. Crossovers in regions B, C, I and J produced DNA sequences differing only by silent nucleotide changes.

B.0203

0141484

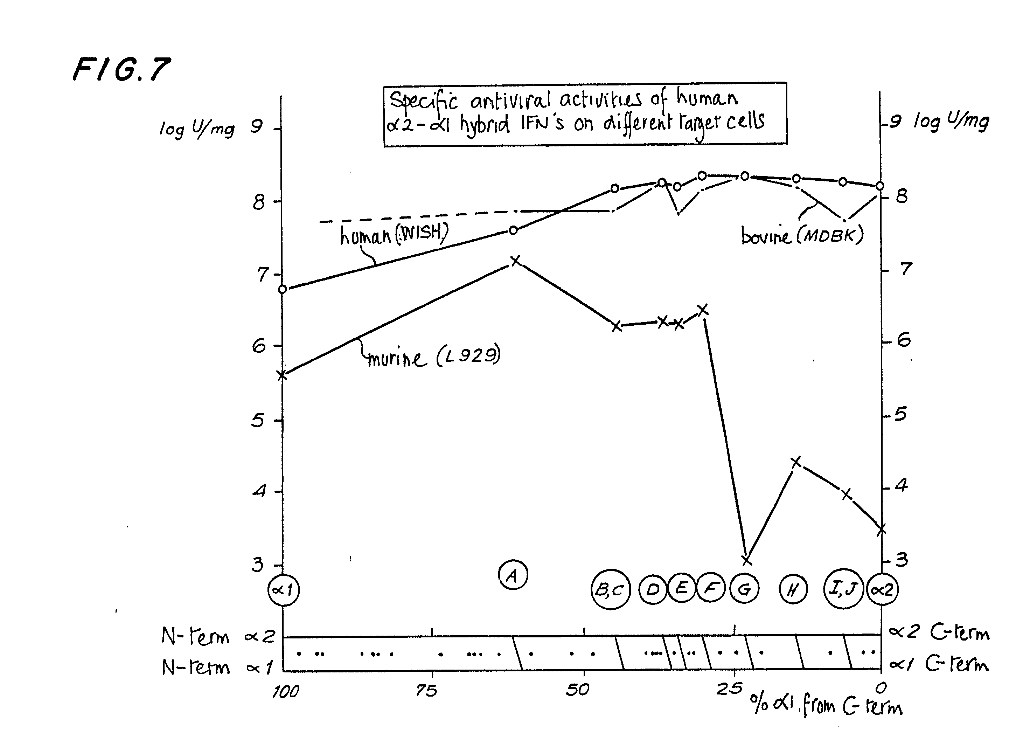

We employed these recombinant α2-α1 DNA sequences to transform host cells and to produce the hybrid interferons encoded by them. We then analyzed the antiviral activities of crude extracts of these hybrid interferons on human (WISH) cells, murine (L929) cells and bovine (MDBK) cells. The antiviral activities of these hybrid interferons, as well as those of the parental IFN-α1 and IFN-α2, are displayed in Figure 7.

The utility of the methods of this invention to produce interferons having different properties than their parents is exemplified by the dramatic drop observed in antiviral·activity on mouse cells as the COOH-terminal α1 segment in the hybrid interferon is shortened beyond 25%.

It should of course be understood tht other IFN-α hybrids may be equally well produced by the methods of this invention. For example, the method chosen for preparation of the linear DNA sequence employed above to form the hybrid DNA sequences only allowed production of hybrids with the amino terminal of α2 and the carboxy terminal of α1, with the crossover region downstream of the BglII site of α1. However, other restriction and digestion patterns allow us to obtain crossover in the parental DNA sequences at any position or between two other preselected positions. Some of these alternative methods are illustrated in Examples II through V, below.

## Example II

In this example we provide one alternative method for preparing an α2-α1 hybrid DNA sequence with crossover at any position.

In this method, we restrict plasmid pM21 with SalI and PstI and isolate the fragment that contains the IFN-α2 coding region. An XbaI linker is then introduced upstream of the IFN-α1 gene in pM11kan to generate pM11kan-Xba. For this construction a 1.4 Kb SalI-EcoRI 1.4 Kb fragment from plasmid pGI was employed because it contained an Xba restriction site upstream of the α1

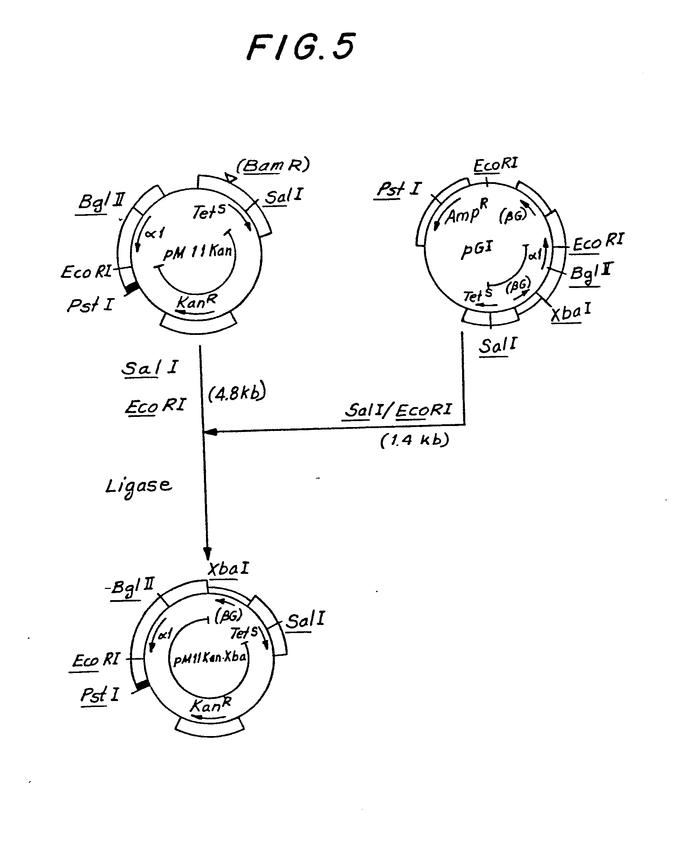

coding sequence. See, e.g., U. Weidle and C. Weissmann, "The 5'-Flanking Region Of A Human IFN-α Gene Mediates Viral Induction Of Transcription," Nature, 303, pp. 442-46 (1983). This construction is depicted in Figure 5. We then restrict pM11kan-Xba with SalI and XbaI and isolate the SalI-XbaI fragment that contains the IFN-α1 coding region. Other restriction sites may be equally well employed to generate the α1 or α2-containing fragments in this Example without departing from the scope of this invention. E.g., the SalI-PstI fragment of pM11kan maybe employed to generate an α1-containing fragment.

Ligation of these two fragments produces a DNA sequence, or more usually a concatemer thereof, comprising in sequence the α2 coding region, an intact replicon from pBR322, and the α1 coding region. The DNA sequence also contains the resistance marker for kanamycin and the reconstructed marker for tetracycline resistance.

After transfection of calcium-treated E.coli substantially as described in Example I, that DNA sequence permits the production of a variety of α2-α1 hybrids having the possibility of crossover at any region where the sequences of homology between the coding sequences of α1 and α2 are at least a series of 5 nucleotides, and more preferably at least 8-13 nucleotides.

EXAMPLE III

In this example, we provide another embodiment of a method for preparing an α2-α1 hybrid DNA sequence with the crossover in the first third of the coding regions. In this method, we restrict plasmid pM21 with SalI and BglII and isolate the SalI-BglII fragment, containing the coding region for the amino terminal end of IFN-α2. We also restrict pM11kan-Xba with XbaI and PstI and isolate the XbaI-PstI fragment, containing the coding region for IFN-α1. Ligation of these fragments produces a DNA sequence, or concatemer thereof, comprising in sequence about the first third of the α2 coding region, an intact replicon from pBR322, and the α1 coding region. Again, other restriction sites may equally well be employed to generate these fragments. E.g., the SalI-PstI fragment of pM11kan maybe employed to generate an α1-containing fragment.

After transfection of calcium-treated E.coli, substantially as described in Example I, that DNA sequence permits the production of a variety of α2-α1 hybrids having the possibility of crossover in the first third of the α2 coding region, where the required homology with the α1 coding region exists.

## EXAMPLE IV

In this example, we provide a method for preparing an α2-α1 hybrid DNA sequence with the crossover in the middle third of the coding regions. In this method, we restrict plasmid pM21 with SalI and PvuII and isolate the SalI-PvuII fragment, containing the coding region of about the first two-thirds of IFN-α2. We also restrict pM11Kan with SalI and BglII and isolate the SalI-BglII fragment containing the about last two-thirds of the IFN-α1 coding region. Again, other restriction fragments may also be used. E.g., an XbaI-SalI fragment of pM11Kan-Xba maybe used to generate an α1-containing fragment.

Ligation of these fragments produces a DNA molecule, or concatemer thereof, comprising in sequence about the first two-thirds of the α2 coding region, an intact replicon from pBR322, and about the last two-thirds of the α1 coding region.

After transfection of calcium-treated E.coli, substantially as described in Example I, that DNA sequence permits the production of a variety of α2-α1 hybrids having the possibility of crossover in the about middle one-third of the α2 coding region, where the required homology with the α2 coding region exists.

0141484

## EXAMPLE V

In this example, we provide a method for preparing an α1-α2 hybrid DNA sequence with the crossover in the carboxy-terminal end of the α1 coding region. In this method, we restrict plasmid pM21 with SalI and BglII and isolate the SalI-BglII fragment containing the coding region for the COOH-terminus of α2. We also restrict pM11Kan with SalI and PstI and isolate the shorter SalI-PstI fragment, containing the entire coding region for α1. Ligation of these fragments produces a DNA sequence, or concatemer thereof, comprising in sequence the COOH-terminal end of the α2 coding region, an intact replicon from pBR322, and the entire α1 coding region.

After transfection of calcium-treated E.coli, substantially as described above, that DNA sequence permits the production of a variety of α1-α2 hybrids having the possibility of crossover in the carboxy terminal end of the α1 coding region, where the required homology exists with the α2 coding region.

The antibiotic selection method does not apply to this example.

## EXAMPLE VI

In Examples I-V the circularization step of the linear DNA sequence was done in vivo. This is the most preferred method of this invention. However, circularization may also be effected in vitro and only heteroduplex repair left to take place in vivo. This example illustrates this less preferred method of this invention.

We digested pM11Kan with BglII to linearize it at position 189. We also partially digested pM21 with BglII (about 60-70% of the full length linearized molecules ended at the downstream BglII site (position 446)). We then digested each of the linearized plasmid preparations with $T_4$-DNA polymerase to convert the terminal 300-400 nucleotides of each linear DNA sequence into

0141484

single-stranded form. After mixing the two sequences in about equal proportions, we allowed the mixture to anneal and filled in any remaining gaps with DNA polymerase. We then cleaved the resulting products with SalI and circularized them in the presence of ligase using standard conditions.

We then transfected calcium-treated E.coli HB101 (rec A⁻), E.coli 803 (rec+) or E.coli SK1592 (rec A⁺ sbcB) with the resulting DNA. We obtained colonies resistant to both tetracycline and kanamycin from each strain, but among the 21 clones that we analyzed further, only 1 (an HB101 transfectant) did not have a deletion or an otherwise rearranged sequence. This clone had 2 PvuII sites, 1 EcoRI site and its region of recombination was in Region C (Figure 6). Therefore, while the in vivo process is more efficient and hence preferred, the in vitro process also permits the production of the hybrid DNAs of this invention.

## EXAMPLE VII

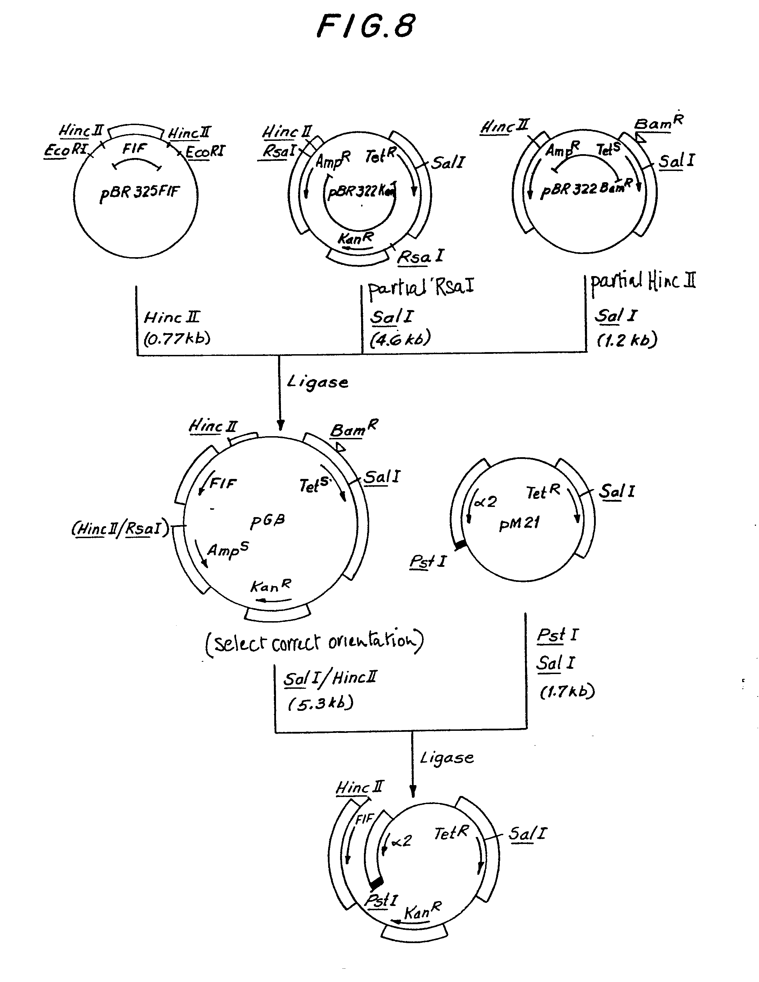

In this example, we prepared recombinants of IFN-α2 and IFN-β. A schematic outline of this method is depicted in Figure 8. The DNA sequence coding for IFN-β is described, for example, in R. Derynck et al., "Isolation And Structure Of A Human Fibroblast Interferon Gene", Nature, 285, pp. 542-46 (1980). See also European patent application 41313 and the clones deposited and made available in connection therewith.

We prepared plasmid pGB (Figure 8) by combining three DNA fragments: (1) the 0.77 Kb HincII fragment of plasmid pBR325 FIF (a gift of W. Fiers), this fragment contains the coding region for IFN-β; (2) the 4.6 Kb partial RsaI-SalI fragment of pBR322kan (described above); and (3) the 1.2 Kb partial HincII-SalI fragment of pBR322 Bam$^R$ (described above) in the presence of ligase. We then used that plasmid to prepare transformants and selected positive clones by resistance to ampicillin, kanamycin and

- L / -

tetracycline, as described above. From these positive clones, we selected one clone characterized by a plasmid having the inserts in the orientation depicted in Figure 8. We designated this plasmid pGB.

We next restricted pGB with SalI and HincII and isolated the 5.3 Kb SalI-HincII fragment that contains the IFN-β coding region. We also restricted pM21 with PstI and SalI and isolated the 1.7 Kb PstI-SalI fragment that contains the IFN-α2 coding region. We then combined these fragments in the presence of ligase.

The resulting DNA fragment (Figure 8) comprised in sequence the DNA sequence encoding IFN-β, the DNA sequences encoding kanamycin resistance and tetracycline resistance and the DNA sequence encoding IFN-α2. Moreover, the two IFN coding sequences were in the same translational orientation relative to each other so as to permit parallel overlap of homologous sequences to form hybrids according to the methods of this invention.

In order to circulize the above-described DNA fragment and to form the desired hybrid DNA sequences, we transfected it into E.Coli SK 1592 and selected positive clones as before.

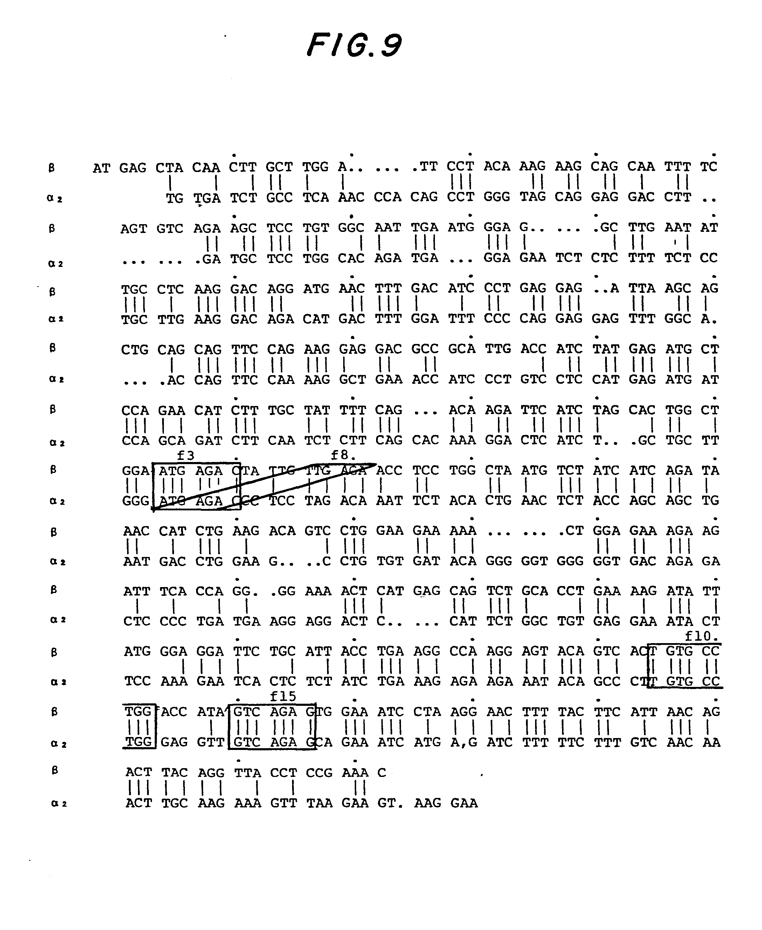

We obtained hybrids from four crossover regions: F3, F8, F10 and F15. See Figure 9. Each had the 5' part of IFN-α2 and the 3' part of IFN-β. Recombinants in crossover regions F3, F10 and F15 were "in phase", i.e. they occur at homologous positions. Recombinants in crossover region F8 are "illegitimate" recombinants because of the repeat TGAGA homology at two positions in the IFN-β coding region. See Figure 9.

We used these α2-β recombinants to transform host cells and to produce the α2-β hybrid interferons encoded by them. We then assayed the antiviral activity of the α2-β hybrid interferons on both human and bovine cells using crude extracts and conventional methods. The results are depicted in Table II.

## TABLE II

| Cells | Hybrid Interferons | | | |
|---|---|---|---|---|
| | F3 | F8 | F10 | F15 |
| Human (WISH) Cells | - | - | - | 150 |
| Bovine (MDBK) Cells | - | - | 50 | 2500 |

Therefore, hybrid α2-β interferons from crossover regions F10 and F15 have antiviral activity. Moreover, the ratio of such activity on human and bovine cells is about 1:15. As a comparison, IFN-α2 has a ratio of antiviral activity on human and bovine cells of about 1:1.

Again, it should be understood that the above example is merely illustrative. Other regions of crossover between IFN-α2 and IFN-β may be utilized and other hybrid α-β or β-α interferons may be prepared in accordance with the teachings of this invention.

## EXAMPLE VIII

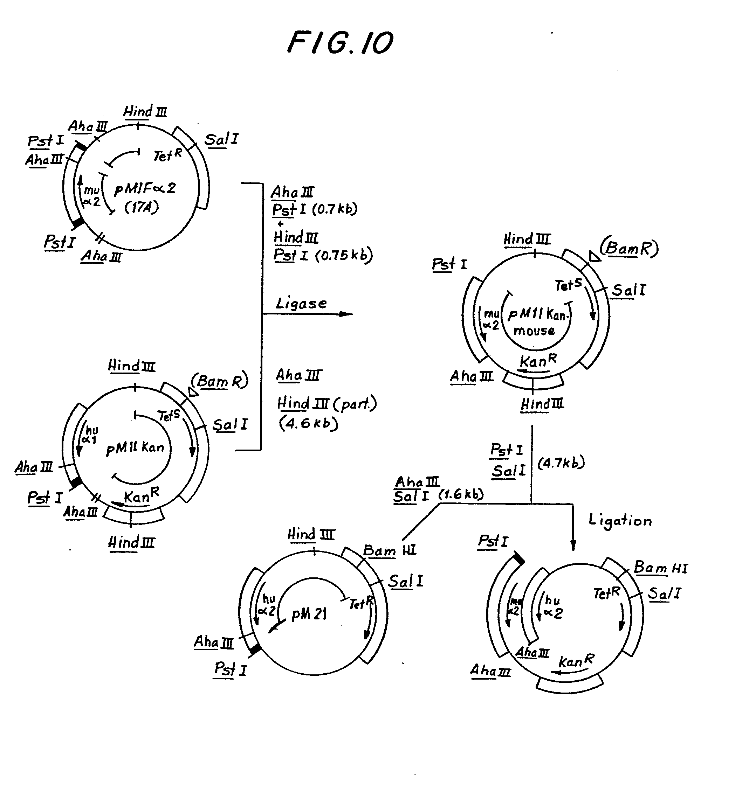

In this example, we prepare recombinants of human IFN-α2 and mouse IFN-α2. A schematic outline of this method is depicted in Figure 10.

As depicted in Figure 10, we prepare plasmid pM11Kan-mouse by ligation of three DNA fragments: (1) the 0.7 Kb AhaIII-PstI fragment of pMIFα2 [G.D. Shaw et al., Nucleic Acids Res., 11, pp. 555-73 (1983)]; (2) the 0.75 kb HindIII-PstI fragment of pMIFα2; (3) the 4.6 Kb AhaIII-HindIII (partial) fragment of pM11Kan. We then restrict pM11Kan-mouse with PstI and SalI to prepare a 4.7 Kb PstI-SalI fragment containing the mouse IFN-α2 coding region. We next combine this fragment with the 1.6 Kb AhaIII-SalI fragment from pM21 that contains the amino-terminal portion of the human IFN-α2 coding region. Upon transformation into host cells as before, we are able to generate various recombinant human α2-mouse α2 DNA sequences that are useful in producing human-mouse hybrid interferons.  Again, it should be understood that other
restriction sites and IFN-α genes may be used to generate
such human-mouse hybrid DNA sequences and interferons in
accordance with the methods of this invention.

Claims:

1. A method for preparing a hybrid DNA sequence comprising the steps of:

(a) preparing a DNA fragment, or concatemer thereof, said fragment comprising in sequence one of the parental DNA sequences from which the hybrid DNA sequence is to be derived, an intact replicon such that the DNA fragment may be replicated in a host cell, and the other parental DNA sequence from which the hybrid DNA sequence is to be derived, the two parental DNA sequences having a sequence of at least three nucleotides in common in the location where hybrid DNA sequence formation is sought and the direction of translation of the coding sequences in the two parental sequences being the same relative to each other in the DNA fragment.

(b) selecting host cells that have been transfected with the desired hybrid DNA sequence and isolating said hybrid DNA sequence from them.

2. The method according to claim 1, wherein the parental DNA sequences have homologous sequences of at least 5 nucleotides.

3. The method according to claim 1, wherein the parental DNA sequences have homologous sequences of at least 8 nucleotides.

4. The method according to claim 1, wherein the parental DNA sequences have homologous sequences of at least 13 nucleotides.

5. The method of any one of claims 1 to 4, wherein the DNA fragment also comprises at least one antibiotic resistance marker.

6. The method of claim 5, wherein the DNA fragment comprises a different antibiotic resistance marker associated with each of said parental DNA sequences.

7. The method of claim 5, wherein the DNA fragment comprises one antibiotic resistance marker and a portion of a different antibiotic resistance marker associated with one of said parental DNA sequences and the portion of said different antibiotic resistance marker that completes it associated with the other of said parental DNA sequences.

8. A method for preparing a hybrid DNA sequence comprising the steps of:

(a) preparing a DNA fragment, or concatemer thereof, said fragment comprising in sequence one of the parental DNA segments from which the hybrid DNA sequence is to be derived, an intact replicon such that the fragment may be replicated in a host cell, and the other of the parental DNA sequences from which the hybrid DNA sequence is to be derived, the direction of translation of the coding sequences in the two parental sequences being the same relative to each other in the DNA fragment;

(b) forming in one of said parental DNA sequences, a sequence of at least three nucleotides, said five nucleotide sequence being identical to a DNA sequence in said other parental DNA sequence at the location in said other DNA sequence where said hybrid formation is sought;

(c) selecting host cells that have been transfected with the desired hybrid DNA sequence and isolating said hybrid DNA sequences from them.

9. The method of claim 8, wherein a sequence of at least 5 common nucleotides is formed in one of said parental DNA sequences.

10. The method according to claim 8, wherein a sequence of at least 13 common nucleotides is formed in one of said parental DNA sequences.

11. The method of any one of claims 8 to 10, wherein said step of forming said common nucleotide sequence in one of said parental DNA sequences takes place before the step of preparing said linear DNA fragment.

12. The method of any one of claims 8 to 11, wherein the DNA fragment also comprises at least one antibiotic resistance marker.

13. The method of claim 12, wherein the DNA fragment comprises a different antibiotic resistance marker associated with each of said parental DNA sequences.

14. The method of claim 12, wherein the DNA fragment comprises one antibiotic resistance marker and a portion of a different antibiotic resistance marker associated with one of said parental DNA sequences and the portion of said different antibiotic resistance marker that complete it associated with the other of said parental DNA sequences.

15. The method of claim 1 or 8, wherein said DNA fragment is rendered partially single stranded at its ends and annealed before the step of transfecting said host with the fragment.

16. The method of any one of the preceding claims, wherein said parental DNA sequences are selected from the group consisting of DNA sequences encoding human α interferons, animal α interferons, human β interferons, animal β interferons, human γ interferons and animal γ interferons.

17. The method of any one of claims 1-16, wherein said parental DNA sequences are selected from the group consisting of DNA sequences encoding human and animal lymphokines.

18. The method of any one of claims 1-16, wherein said parental DNA sequences are selected from the group consisting of DNA sequences encoding one or more of the antigens of foot and mouth disease virus.

19. The method according to claim 6, 7, 13 or 14, wherein said step of selecting said host cells comprises the steps of growing the transformed host cells on agar plates containing both antibiotics.

20. A hybrid DNA sequence prepared by the method of any one of the preceding claims.

21. A hybrid DNA sequence comprising at least two connected sequences of nucleotides, each connected DNA sequence being derived from a different parental DNA sequence, the first sequence of nucleotides being connected to the second sequence of nucleotides in the same reading frame so as to maintain constant that reading frame in each sequence through a DNA sequence of at least three nucleotides that was common to both parental DNA sequences at the location where hybrid formation was sought.

22. The hybrid DNA sequence according to claim 21, wherein these are at least five nucleotides that were common to both parental DNA sequences.

23. The hybrid DNA sequence according to claim 21 or 22, wherein said parental DNA sequences are selected from the group consisting of DNA sequences encoding human α interferons, animal α interferons, human γ interferons, animal β interferons, human γ interferons and animal γ interferons.

24. The hybrid DNA sequence according to claim 21 or 22, wherein said parental DNA sequences are selected from the group consisting of DNA sequences encoding human and animal lymphokines.

25. The hybrid DNA sequence according to claim 21 or 22, wherein said parental DNA sequences are selected from the group consisting of DNA sequences encoding one or more of the antigens of foot and mouth disease virus.

26. A hybrid polypeptide produced by a method comprising the steps of culturing a host transformed with a hybrid DNA sequence selected from the group consisting of hybrid DNA sequences prepared by the methods of any one of claims 1 to 20 and the hybrid DNA sequences of any one of claims 21 to 25 and collecting said polypeptide.

27. A hybrid polypeptide comprising at least two connected sequences of amino acids, each connected amino acid sequence being derived from a different parental polypeptide, the first sequence of amino acids being connected to the second sequence of amino acids through an amino acid sequence of at least one amino acid that was common to both parental polypeptides at the location where hybrid formation was sought.

28.    The hybrid polypeptide according to claim 27, wherein at least two amino acids were common to both parental polypeptides at the location where hybrid formation was sought.

FIG.1

FIG.2

# FIG.3

4

0141484

FIG.4

# FIG. 5

FIGURE 6A

<pre>
              1          10          20          30          40          50
              |          |           |           |           |           |
IFN α2    TGT GAT CTG CCT CAA ACC CAC AGC CTG GGT AGC AGG AGG ACC TTG ATG CTC CTG
          Cys Asp Leu Pro Gln Thr His Ser Leu Gly Ser Arg Arg Thr Leu Met Leu Leu


              1                                  10

          Cys Asp Leu Pro Glu Thr His Ser Leu Asp Asn Arg Arg Thr Leu Met Leu Leu
IFN α1    TGT GAT CTC CCT GAG ACC CAC AGC CTG GAT AAC AGG AGG ACC TTG ATG CTC CTG
              1          10          20          30          40          50


                  60          70          80          90          100         110         120                 130         140
                  |           |           |           |           |           |           |                   |           |
α2        GCA CAG ATG AGG AGA ATC TCT CTT TTC TCC TGC TTG AAG GAC AGA CAT GAC TTT GGA TTT CCC CAG GAG GAG TTT --- GGC AAC CAG TTC
          Ala Gln Met Arg Arg Ile Ser Leu Phe Ser Cys Leu Lys Asp Arg His Asp Phe Gly Phe Pro Gln Glu Glu Phe --- Gly Asn Gln Phe


              20                                  30                                          40
          Ala Gln Met Ser Aug Ile Ser Pro Ser Ser Cys Leu Met Asp Arg His Asp Phe Gly Phe Pro Gln Glu Glu Phe Asp Gly Asn Gln Phe
α1        GCA CAA ATG AGC AGA ATC TCT CCT TCC TCC TGT CTG ATG GAC AGA CAT GAC TTT GGA TTT CCC CAG GAG GAG TTT GAT GGC AAC CAG TTC
              60          70          80          90          100         110         120                 130         140


                                                                       ┌──── A ────┐
                  150         160         170         180             190           200         210         220         230
                  |           |           |           |               |             |           |           |           |
α2        CAA AAG GCT GAA ACC ATC CCT GTC CTC CAT GAG ATG ATC CAG CAG ATC TTC AAT CTC TTC AGC ACA AAG GAC TCA TCT GCT GCT TGG GAT
          Gln Lys Ala Glu Thr Ile Pro Val Leu His Glu Met Ile Gln Gln Ile Phe Asn Leu Phe Ser Thr Lys Asp Ser Ser Ala·Ala Trp Asp
                  50                                          60          Bgl II                             70
</pre>

FIGURE 6B

α1

50                                              60                    '                    70
GLN LYS ALA PRO ALA ILE SER VAL LEU HIS GLU LEU ILE GLN GLN ILE PHE ASN LEU PHE THR THR LYS ASP SER SER ALA ALA TRP ASP
CAG AAG GCT CCA GCC ATC TCT GTC CTC CAT GAG CTG ATC CAG CAG ATC TTC AAC CTC TTT ACC ACA AAA GAT TCA TCT GCT GCT TGG GAT
    150         160         170         180    B    190         200    C    210         220    D    230

α2

    240         250    ┌260         270         280         290         300         310 ┐    320
GAG ACC CTC CTA GAC AAA TTC TAC ACT GAA CTC TAC CAG CAG CTG AAT GAC CTG GAA GCC TGT GTG ATA CAG GGG GTG GGG GTG ACA GAG
GLU THR LEU LEU ASP LYS PHE TYR THR GLU LEU TYR GLN GLN LEU ASN ASP LEU GLU ALA CYS VAL ILE GLN GLY VAL GLY VAL THR GLU
        80                        90                                        100
                                    PVY II

α1

    80                        90                                        100
GLU ASP LEU LEU ASP LYS PHE CYS THR GLU LEU TYR GLN GLN LEU ASN ASP LEU GLU ALA CYS VAL MET GLN GLU GLU ARG VAL GLY GLU
GAG GAC CTC CTA GAC AAA TTC TGC ACC GAA CTC TAC CAG CAG CTG AAT GAC TTG GAA GCC TGT GTG ATG CAG GAG GAG AGG GTG GGA GAA
    240         250         260         270         280         290         300         310         320

α2

    E                    F
┌    330 ┐    ┌ 340 ┐ 350         360         370┌    380         390┐┌    400         410┐
ACT CCC CTG ATG AAG GAG GAC TCC ATT CTG GCT GTG AGG AAA TAC TTC CAA AGA ATC ACT CTC TAT CTG AAA GAG AAG AAA TAC AGC CCT
THR PRO LEU MET LYS GLU ASP SER ILE LEU ALA VAL ARG LYS THR PHE GLN ARG ILE THR LEU TYR LEU LYS GLU LYS LYS TYR SER PRO
    110                        120                                        130

014184

FIGURE 6C

```
        110                                              120                                    130
       Thr Pro Leu Met Asn Ala Asp Ser Ile Leu Ala Val Lys Lys Thr Phe Arg Arg Ile Thr Leu Tyr Leu Thr Glu Lys Lys Tyr Ser Pro
α1     ACT CCC CTG ATG AAT GCG GAC TCC ATC TTG GCT GTG AAG AAA TAC TTC CGA AGA ATC ACT CTC TAT CTG ACA GAG AAG AAA TAC AGC CCT
        330         340       350         360        370        380         390          400         410
```
```
                          H
                          420         430         440         450 |     ||460       470         |480       490         500
α2     TGT GCC TGG GAG GTT GTC AGA GCA GAA ATC ATG AGA TCT, TTT TCT TTG TCA ACA AAC TTG CAA GAA AGT TTA AGA AGT AAG GAA TGA AAA
       Cys Ala Trp Glu Val Val Arg Ala Glu Ile Met Arg Ser Phe Ser Leu Ser Thr Asn Leu Gln Glu Ser Leu Arg Ser Lys Glu
              140                                    150                                        160            165
              140                              Bgl II                                           160            166
                                              150
```
```
       Cys Ala Trp Glu Val Val Arg Ala Glu Ile Met Arg Ser Leu Ser Leu Ser Thr Asn Leu Gln Glu Arg Leu Arg Arg Lys Glu
α1     TGT GCC TGG GAG GTT GTC AGA GCA GAA ATC ATG AGA TCC CTC TCT TTA TCA ACA AAC TTG CAA GAA AGA TTA AGG AGG AAG GAA TAA CAT
        420         430         440         450         460         470         480         490         500
```
```
                          K
              510      520       530         540         550          560         570         580
α2     CTG GTT CAA CAT GGA AAT GAT TTT CAT TGA TTC GTA TGC CAG CTC ACC TTT TTA TGA --T CTG CCA TTT CAA AGA CTC ATG TTT CTG CTA
```
```
                                                                          Eco RI
1      CTG GTC CAA CAT GAA AAC AAT TCT TAT TGA CTC ATA CAC CAG GTC ACG CTT TCA TGA ATT CTG TCA TTT CAA AGA CTC TCA CCC CTG CTA
        510         520         530         540         550          560         570         580         590
```

014484

# FIG.7

Specific antiviral activities of human α2-α1 hybrid IFN's on different target cells

log U/mg (left axis, values 3–9)

log U/mg (right axis, values 3–9)

human (WISH)

bovine (MDBK)

murine (L929)

α1    A    B,c   D   E   F   G   H   I,J   α2

N-term α2                                    α2 C-term
N-term α1                                    α1 C-term

100        75        50        25        0

% α1 from C-term

9

0141484

## FIG.8

# FIG.9

```
β    AT GAG CTA CAA CTT GCT TGG A.. ... .TT CCT ACA AAG AAG CAG CAA TTT TC
         |   |   |  ||   |   |           |||       ||  ||   ||  |   ||
α2          TG TGA TCT GCC TCA AAC CCA CAG CCT GGG TAG CAG GAG GAC CTT ..

β    AGT GTC AGA AGC TCC TGT GGC AAT TGA ATG GGA G.. ... .GC TTG AAT AT
         ||   ||  |||  ||     |   |   |||      ||| |       |  ||   ' |
α2   ... ... .GA TGC TCC TGG CAC AGA TGA ... GGA GAA TCT CTC TTT TCT CC

β    TGC CTC AAG GAC AGG ATG AAC TTT GAC ATC CCT GAG GAG ..A TTA AGC AG
     |||  |   |||  |||  ||       ||  |||  |   |   ||   ||  |||     ||   |
α2   TGC TTG AAG GAC AGA CAT GAC TTT GGA TTT CCC CAG GAG GAG TTT GGC A.

β    CTG CAG CAG TTC CAG AAG GAG GAC GCC GCA TTG ACC ATC TAT GAG ATG CT
         |   |||  |||  ||   |||  |   ||   ||           |  ||   ||  |||  |||  |
α2   ... .AC CAG TTC CAA AAG GCT GAA ACC ATC CCT GTC CTC CAT GAG ATG AT

β    CCA GAA CAT CTT TGC TAT TTT CAG ... ACA AGA TTC ATC TAG CAC TGG CT
     |||  ||   |   ||  |||       ||   |  |||       |   |   ||   ||  |||  |       ||   |
α2   CCA GCA GAT CTT CAA TCT CTT CAG CAC AAA GGA CTC ATC T.. .GC TGC TT

                f3            .         f8.
β    GGA|ATG AGA CTA TTG TTG ACA|ACC TCC TGG CTA ATG TCT ATC ATC AGA TA
     ||  |||  |||  |  |   |   |  |   |   ||   ||  |   |||  |  |  |  |   ||   |
α2   GGG|ATG AGA CCC|TCC TAG ACA AAT TCT ACA CTG AAC TCT ACC AGC AGC TG

β    AAC CAT CTG AAG ACA GTC CTG GAA GAA AAA ... ... .CT GGA GAA AGA AG
     ||   |   |||  |          ||   ||   |||            ||   ||  |||
α2   AAT GAC CTG GAA G.. ..C CTG TGT GAT ACA GGG GGT GGG GGT GAC AGA GA

β    ATT TCA CCA GG. .GG AAA ACT CAT GAG CAG TCT GCA CCT GAA AAG ATA TT
     |   |   |   |       |   |||  |   ||   |||  |   |   |   |||  |   |
                                                                    f10.
α2   CTC CCC TGA TGA AGG AGG ACT C.. ... CAT TCT GGC TGT GAG GAA ATA CT

β    ATG GGA GGA TTC TGC ATT ACC TGA AGG CCA AGG AGT ACA GTC AC|T GTG CC
     |   ||||  |   |    |   |   ||  |||  ||   |   |||  |  |  |||  |   |||  |||
α2   TCC AAA GAA TCA CTC TCT ATC TGA AAG AGA AGA AAT ACA GCC CT|T GTG CC

         f15
β    |TGG|ACC ATA|GTC AGA G|TG GAA ATC CTA AGG AAC TTT TAC TTC ATT AAC AG
     |||  |   |    |||  |||  |    |||  |||  |   |  |  |   |||  |  |   |   |||  |
α2   |TGG|GAG GTT|GTC AGA G|CA GAA ATC ATG A,G ATC TTT TTC TTT GTC AAC AA

β    ACT TAC AGG TTA CCT CCG AAA C
     |||  |  |  |   |   |      |   |           ||
α2   ACT TGC AAG AAA GTT TAA GAA GT. AAG GAA
```

FIG.10